# EUROPEAN PATENT APPLICATION

(11) **EP 0 606 044 A1**
(43) Date of publication of application: **13.07.1994**
(21) Application number: 93810835.4
(22) Date of filing: 29.11.1993
(51) Int. Cl.: C07D 313/00, A61K 31/365, C12P 17/08

(54) **Lactones compounds useful as pharmaceuticals**

(30) Priority: 04.12.1992 GB 9225396
(71) Applicant: SANDOZ LTD., CH-4002 Basel (CH); SANDOZ-PATENT-GMBH, D-79539 Lörrach (DE); SANDOZ-ERFINDUNGEN Verwaltungsgesellschaft m.b.H., A-1235 Wien (AT)
(72) Inventor: Dreyfuss, Michael Morris, CH-4054 Basle (CH); Leutwiler, Albert, CH-3178 Bösingen (CH); MacKenzie, Andrew Roland, CH-3251 Wengi b. Büren (CH); Schnyder, Jörg, CH-3006 Bern (CH); Traber, René Paul, CH-4052 Basle (CH); Mattes, Henri, F-68200 Brunstadt (FR)

(57) **Abstract**

Novel compounds of formula I 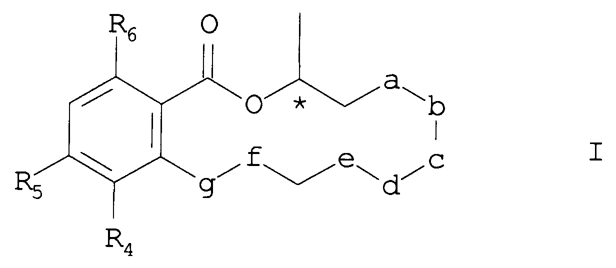
wherein R₄, R₅, R₆, -a-b-, c, -d-e- and -f-g- are as defined are cytokine release inhibitors and IL-1 antagonists and are thus indicated for treatment of disorders with an aetiology associated with or comprising excessive cytokine release, particularly IL-1β release, including a wide variety of inflammatory states and diseases such as RA, osteoarthritis, septic shock, psoriasis, atherosclerosis, inflammatory bowel disease, Crohn's disease and asthma. Related known compounds Zearalenone and radicicol and derivatives thereof have also been found to have cytokine release inhibitor properties and have similar pharmaceutical applications.

## Description

This invention relates to compounds useful as a cytokine release inhibitors and IL-1 antagonists.

The invention provides the novel compounds of formula I 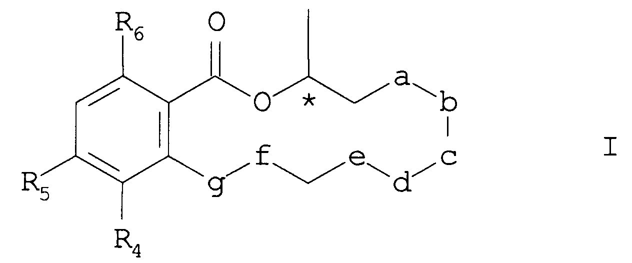
in which R₄ and R₆ are the same or different and are H, OH, C₁₋₄ alkoxy or C₁₋₄alkyl COO-,
R₅ is OH, C₁₋₄alkoxy or C₁₋₄alkyl COO,
one of -a-b- or -d-e- is -CHR₇-CHR₈- and the other is cis- or trans- -CR₇=CR₈-, wherein R₇ and R₈ are the same or different and are H, OH, C₁₋₄ alkoxy or C₁₋₄alkyl COO-,
c is CH-OH or C=O and
-f-g- is -CH₂-CH₂- or cis- or trans- -CH=CH-
provided that, when R₄ is H, R₆ is OH and -f-g- is trans- -CH=CH-; 1. R₅ is not OH when -a-b- is -CH₂-CH₂-, c is C=O and -d-e- is -CH₂-CH₂-, or 2. R₅ is not methoxy when -a-b- is -CH₂-CH₂- or cis- -CH=CH- and c is C=O or CH-OH and -d-e- is -CHOH-CHOH,
in free form or base salt form or in the form of a physiologically-hydrolysable and -acceptable ester and wherein the asymmetric carbon marked * and the atoms a and/or b or d and/or e, when these are asymmetric carbon atoms have the R- or S-configuration or the compound comprises any mixture of the optical isomers thereof.

Preferably R₄ and R₆ are the same or different and are H, -OH, MeO- or Me-COO-. Preferably R5 is -OH, MeO- or MeCOO-. More preferably R₄ is H or MeO; R₅ is MeO, and R₆ is OH or MeO.

Preferably -a-b- is cis- or trans- -CR₇'=CR₈'-, wherein R₇' and R₈' are the same or different and are H, OH, MeO- or Me-COO-. More preferably -a-b- is cis- or especially trans- -CH=CH-.

Preferably -d-e- is -CHR₇'-CHR₈'-, wherein R₇' and R₈' are as defined above. More preferably -d-e- is -CH₂-CH₂- or especially -CHOH-CHOH-, wherein the OH groups may be in free or protected form.

Most preferably -f-g- is trans- -CH=CH-.

Preferably the asymmetric carbon atoms of the compounds of the invention all have the S-configuration.

In particular embodiments the invention provides compounds of formula I in which R₄ is H or methoxy, R₅ is methoxy, R₆ is OH, -a-b- is cis- or trans- -CH=CH-, c is CHOH or C=O, -d-e- is -CHOH-CHOH- and -f-g- is trans- -CH=CH-; provided that when -a-b- is cis -CH=CH-, then R₄ is methoxy and c is C=O in free form or base salt form or in the form of a physiologically-hydrolysable and -acceptable ester.

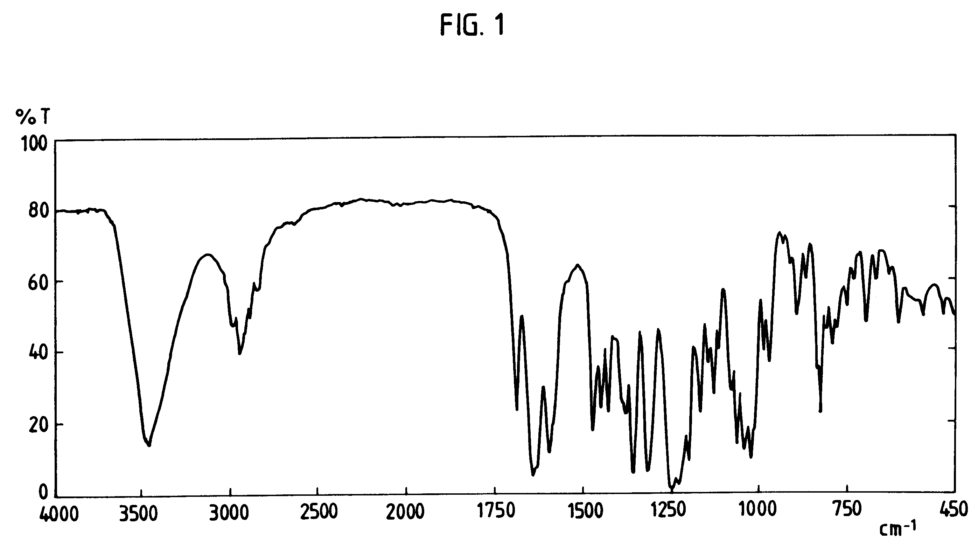

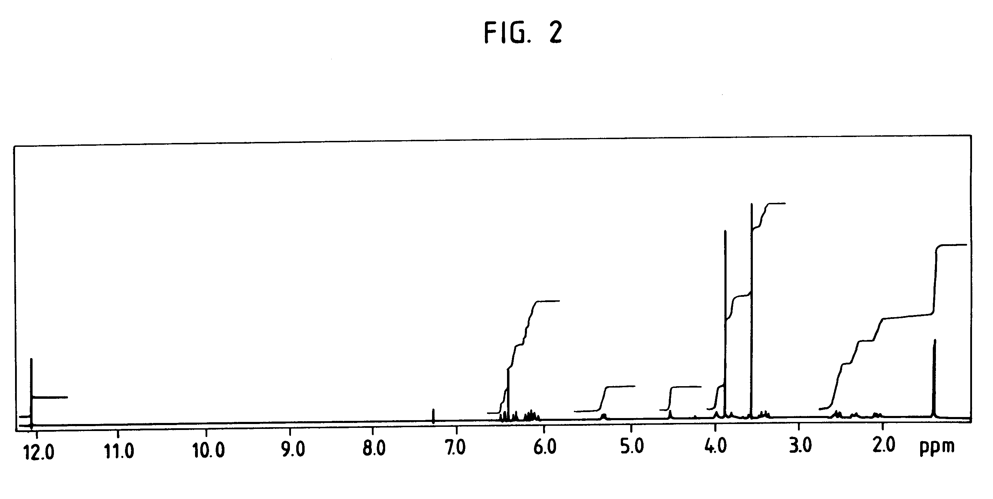

Formula I', which is formula I in which R₄ and R₅ are methoxy, R₆ is OH, -a-b- is cis- -CH=CH-, c is C=O, -d-e- is -CHOH-CHOH-, -f-g- is trans- -CH=CH- and the asymmetric carbon atoms marked * all have the S-configuration 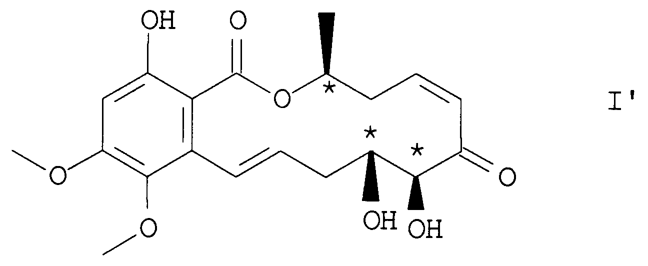
is the structure which has been assigned to the novel metabolite designated 87-250904-F1 and having the following characteristics:
White needles (from methanol/water 1:1), m.p. 173-174°C,
[α]_{D}²⁵ = -43.6°(MeOH, c = 0.76)
Mass spectrum (FAB) : m/e = 393 (MH⁺)
IR spectrum in KBr : see Fig. 1
Proton NMR in CDCl₃, 360 MHz with TMS as internal standard, see Fig. 2
Solubility: Almost insoluble in water, readily soluble in methanol, DMSO, chloroform.
- HPLC :: Column : LiChrospher 100 RP-8 (5µm) (LiChroCART 125-4, Merck)
Mobile phase: acetonitrile/water/orthophosphoric acid 350:650:0.175 (by vol.)
Flow rate: 1.0 ml/min
Detection: UV 210 nm
Retention time: 3.8 min.

The compounds of formula I are novel compounds which belong to the same class of compounds as zearalenone, which is the compound of formula II 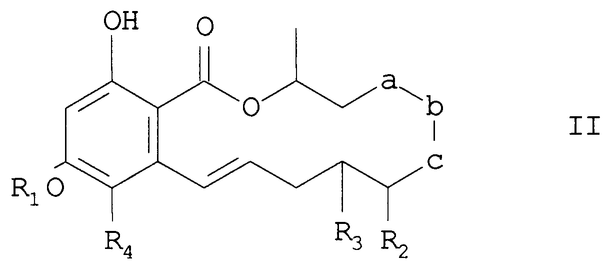
in which R₁ = R₂ = R₃ = R₄ = H, -a-b- = -CH₂-CH₂- , and c = C=O. Zearalenone is known to have anabolic effects. The compounds of formula II in which R₁ is methyl, R₂ = R₃ = OH, R₄ = H, -a-b- = -CH₂-CH₂- or cis- -CH=CH- and c = C=O or CH-OH are described by Ellestad et al, J. Org. Chem. **43** 2339 (1978), where they are stated to have "no particularly interesting activities" and it is speculated that the presence of the phenolic ether (i.e. the methoxy group on the benzene ring) may be responsible for this lack of activity.

In view of this it is surprizing that the compounds of formula I should have any pharmacological activity.

The compounds of formula I are also related to the compound radicicol, which is the compound of formula III 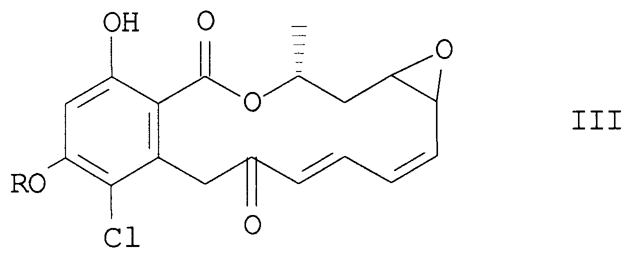
in which R is H. The corresponding methoxy compound in which R is methyl is also known.

Radicicol, a metabolite of Monosporium bonorden, is known to have antibiotic properties [Delmotte, Nature **171** 344 (1953)]

Surprizingly, it has now been found that the compounds described above, namely the novel compounds of Formula I, radicicol, O-methyl radicicol and the zearalenone derivatives described by Ellestad et al also have cytokine release inhibitor properties, in particular, they inhibit the release of IL-1, IL-6 and TNF-α, and also act as functional antagonists of IL-1.

The compound of formula I' may be produced by cultivating a producing microbial strain in a nutrient medium. Preferred micro-organisms are strains of pycnidial imperfect fungi, in particular the strain F/87-250904, which produces the metabolite 87-250904-F1.

This strain has been isolated from an unidentified lichen collected in South Africa, and a viable culture of the strain was deposited on 6 Nov.1991 at the ARS Patent Culture Collection, US Dept. of Agriculture, Northern Regional Research Center, Peoria, Illinois, USA under the provisions of the Budapest Treaty and was given the reference number NRRL 18919. The culture may also be obtained from Sandoz Ltd., Basle, Switzerland.

The fungal strain NRRL 18919 grows on most usual fungal agar media such as 2 % malt extract agar. The temperature range for growth is between approx. 5 and 37°C, the optimal temperature for growth is between approx. 24 and 32°C. On 2% malt extract agar in petri dishes and at 27°C strain NRRL 18919 will form after 10 days incubation colonies 25 to 35 mm in diameter. The colonies appear brown to greenish black to black with moderately developed aerial mycelium. No pronounced diffusable pigments are formed under these conditions. Strain NRRL 18919 can degrade starch and keratin, but not or only to a very limited degree cellulose and chitin.

The pycnidia of strain NRRL 18919 are very variable in shape and size. They range from approx. 30µ large, globose to pyriform with a single ostiolum to irregularly shaped, up to 600µ large pycnidia or pycnidial complexes with several ostioli. The outer pycnidial walls are composed of brown cells. The conidia are hyaline, aseptate, bacilliform, reniform to allantoid, often constricted and 3.0 - 5.5 x 1.0 - 1.7µ large. Conidia are produced within the pycnidia by slender phialides and collect outside the ostioli in a whitish mass. The strain NRRL 18919 can best be accomodated in the genus Phoma Sacc. and fits quite well to the description of Phoma cava Schulzer.

The new strain NRRL 18919 may be cultured at suitable temperatures in various culture media using appropriate nutrients and mineral substances, as aerobic surface or immersion cultures. The invention also provides fermentation broths which are obtained by cultivation of an 87-250904-F1 producing fungal strain, particularly of the strain NRRL 18919. A further aspect of the invention provides a process for the preparation of the compound of Formula I comprising the steps of cultivating an 87-250904-F1-producing fungal strain and isolating the metabolite 87-250904-F1 which is formed.

The fermentation media should contain a utilisable source of carbon and optionally mineral salts and growth factors, all of which can be added in the form of well defined products or as complex mixtures, as are found in biological products of various origins.

In order to produce the new metabolite 87-250904-F1, strains may also be used which are obtained by selection or mutation under the influence of ultra-violet radiation, X-rays or by other means, e.g. by the use of chemical mutagens.

As soon as a sufficient amount of metabolite has accumulated in the culture, it may be concentrated and isolated in conventional manner, for example by extraction and subsequent chromatographic methods.

Compounds of formula I in which a-b is trans- -CH=CH- may be prepared from the corresponding compounds in which a-b is cis- -CH=CH- by isomerization under mild alkaline conditions, for example in solution in pyridine, suitably for 12 - 48 hr at temperatures between 0 and 80°C, preferably at about 50°C.

The compounds of formula I may also be prepared by complete or partial chemical synthesis using conventional synthesis techniques.

Thus in a further aspect the invention provides a process for the preparation of a compound of formula I, in which c is CHOH, which comprises cyclising a compound of formula IV 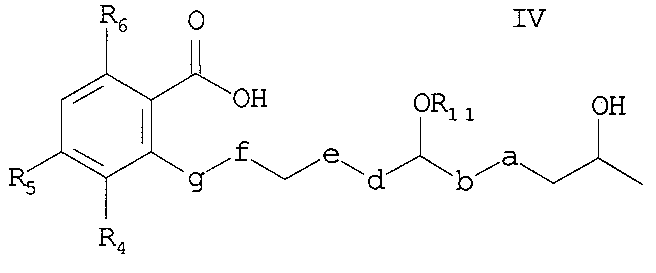
wherein R₄, R₅, R₆, -a-b-, -d-e- and -f-g- are as defined above with the exception that any OH substituents on -a-b- or -d-e- are in suitably protected form, and R₁₁ is H or an OH protecting group;
and removing any OH protecting groups therefrom.

Compounds of formula I in which c is C=O may be prepared from the the cyclised product by oxidation of the CHOH group at c.

The compounds of formula IV wherein -f-g- is trans- -CH=CH- may be prepared by linking compounds of formulae V and VI 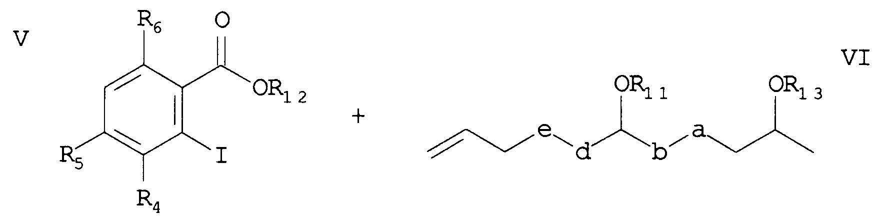
wherein R₄, R₅, R₆, R₁₁, -a-b-, -d-e- and -f-g- are as defined above for formula IV and R₁₂ and R₁₃ are OH protecting groups,
and removing the R₁₂ and R₁₃ are OH protecting groups.

Compounds of formula VI may be prepared by linking an hydroxy protected analogue of 4-hydroxybut-1-yne with hex-1-en-5-one, partially or completely reducing the acetylene bond and, if appropriate, adding an R₁₁ OH protecting group.

The compounds of formula IV wherein -f-g- is -CH₂-CH₂- may be prepared by linking compounds of formulae VII and VIII 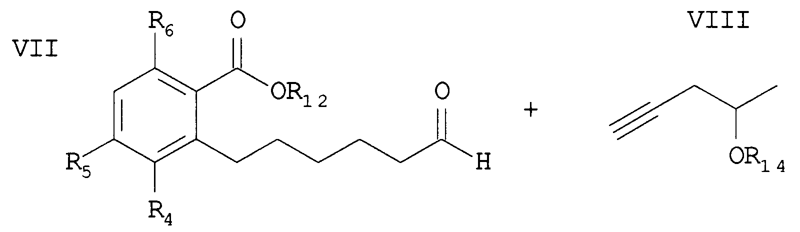
wherein R₄, R₅, R₆, and R₁₂ are as defined above for formulae IV and V and R₁₄ is an OH protecting group,
partially or completely reducing the acetylene bond and if appropriate removing OH protecting groups.

The compound of formula VII may be prepared by linking a compound of formula V as above with an hydroxy protected analogue of 6-hydroxy-hex-1-ene, reducing the alkylene bond corresponding to f-g in formula I, removing the protecting group from the terminal hydroxy group on the C₆ side chain and oxidising this hydroxy group to an aldehyde group.

The processes described above may be carried out using conventional synthesis procedures, reagents and conditions; for instance, as described hereinafter in the Examples.

Preferred compounds in which a-b is trans- -CH=CH- are the compounds of formulae I'', X and XI.

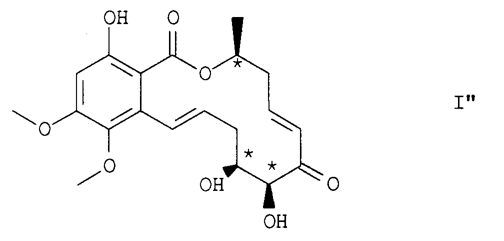

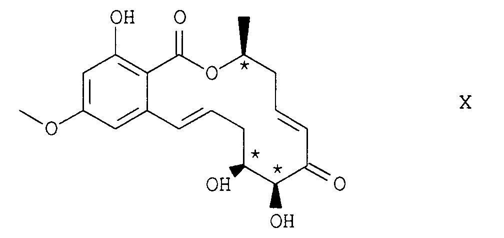

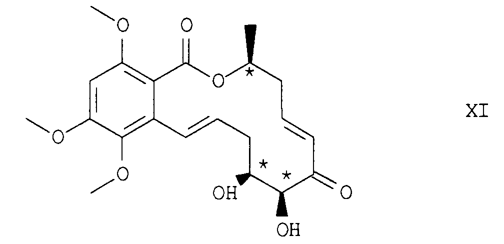

Preferred compounds in which a-b is cis- -CH=CH- are the compounds of formulae I', XII and XIII.

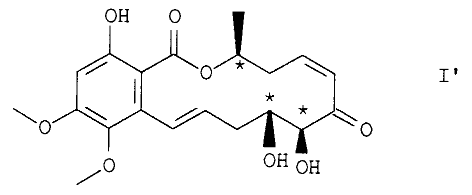

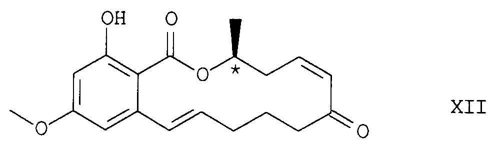

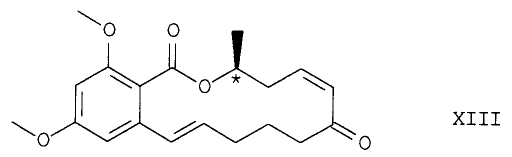

The compounds of the invention have pharmacological properties. In particular, they show cytokine inhibitory effects, acting not only to inhibit the release of IL-1, IL-6 and TNF-α, but also as functional antagonists of IL-1 as indicated in the following in vitro and in vivo test methods:

### 1. Cytokine Release from THP-1 Cells

The THP-1 cell line is generally available and is described by Tsuchiya et al, Int. J. Cancer 26 171-176 (1980). 900 µl THP-1 cells (0.5 x 10⁶ cells) together with 100 U γ-interferon/0.9 ml RPMI 1640 medium (containing 2 mM L-glutamine and 5 % heat-inactivated foetal calf serum) are pipetted into 24 well culture plates. 100 µl of the compound to be tested are then added. After 3 hours at 37°C in 5 %
CO2/95 % air, 10 µl lipopolysaccharide 500 µg/ml is added and the incubation continued for a further 40 hours. Appropriate controls (with and without stimulus, solvent) are also included. The media are then removed and clarified by centrifugation at 1000 g for 10 min. 1.0 ml digitonin 0.01 % is added to the wells to lyse the cells which are loosened by scraping with a rubber policeman and left at 4°C for 10 min. Lactate dehydrogenase measurements are then performed immediately and the samples stored at -20°C until the other determinations can be made. The assays are: IL-1β (medium and lysate), IL-6 (medium), TNF-α (medium), PGE2 (medium and lysate), lactate dehydrogenase (LDH) (medium and lysate) and DNA (lysates) . IL-1β, IL-6 and TNF-α assays are determined using commercially available ELISA kits (Cistron), PGE2 is measured using a standard RIA and DNA fluorimetrically using DAPI (4',6-diamidino-2-phenylindole.2HCl)

In this test, the compounds of the invention inhibit IL-1β ,II-6, TNF-α and PGE2 release at a concentration of about 0.001 to 10 µM. In contrast DNA levels remain substantially unaffected, and the compounds are non-toxic, since LDH release is unchanged.

### 2. Cytokine Release from Human Monocytes

a) Human Monocytes
   Mononuclear cells are obtained from the blood of healthy volunteers via centrifugation and cultivated on tissue culture dishes with the test compound at various concentrations [Schnyder et al., Agents & Actions, **30**, 350-362 (1990)]. The non-adherent lymphocytes are removed after 4 hr. by washing several times. Fresh medium, test compound and LPS (10 µg/ml) as stimulant are added and the monocytes incubated for a further day. The pooled culture media are diluted 1:10 with fresh medium and added to confluent rabbit chondrocytes. Metallo- proteinase (MP) activity in the chondrocyte culture medium is assayed after a further 2 days as described below. Compounds of the invention are active in suppressing monokine release in this test method at a concentration of the order of from 0.001 to 10µM.
b) Determination of IL-1 by the chondrocyte test
   Purified IL-1, recombinant human IL-1-β (rhIL-1) or conditioned media collected from stimulated human monocytes, mouse macrophages or mouse cell line P388D₁, cause characteristic changes in the secretory pattern of chondrocytes. In particular, a latent metalloproteinase (MP) is induced, whilst secretion of plasminogen activator is reduced. The property of the metallo-proteinase or stromelysin has been described in detail [Chin et al., J. Biol. Chem. **260**, 12367 - 12376 (1985)], as has that of the plasminogen activator [Schnyder et al., Analyt. Biochem. **200,** 156-162, 1992]. Dose-response curves using purified or recombinant IL-1 and neutralisation with an antibody to human monocyte IL-1 have shown that this system can be used as a specific and sensitive bioassay for IL-1. Stimulation of the secretion of a latent metalloproteinase by rabbit articular chondrocytes is relatively IL-1-specific, IL-2, TNF-α, recombinant human interferon-α, and -γ, phorbol myristate acetate, Concanavalin A, E-type prostaglandin and indomethacin having no influence [Schnyder and Payne, Brit. J. Rheumatol. **24** (suppl. 1), 128 - 132 (1985); Schnyder et al., J. Immunol. **138**, 496 - 503 (1987)].
   Chondrocytes are harvested and cultured as described [Evequoz et al., Biochem. J. **219**, 667 - 677 (1984)]. Briefly, chondrocytes are released from slices of distal femur articular cartilage from ca. 1.2 kg female New Zealand White rabbits by treatment with proteinases. The washed cells are cultured on 48-well culture plates in DMEM, enriched with 1 % antibiotics, 2 mM glutamine and 10 % heat-inactivated fetal calf serum. After reaching confluency the cells are incubated with 20 µl samples of the test culture media for IL-1 bioassay and made up to a volume of 300 µl Iscove's modified Dulbecco's medium. The supernatant media are collected after 48 h, centrifuged and processed for biochemical analysis.
c) Biochemical Assays
   Metalloproteinase (MP) is measured kinetically by using a 96-well plate Twinreader (Flow Laboratories AG) linked to a personal computer. Ac-Pro-Leu-Gly-S-Leu-Leu-Gly-OC2H5, a synthetic substrate for vertebrate collagenase is used for the determination of MP [Weingarten and Feder, Anal. Biochem. 147, 437-440 (1985)]. 50 µl of the latent MP is activated with 50 µl trypsin (120 µg/ml in 50 mM PIPES pH 6.8, containing 20 mM CaCl2) for 30 min at 37°C, after which time the activities of all serine proteinases are stopped by adding 150 µl Soybean trypsin inhibitor (SBTI; 166 µg/ml in the above buffer). A 50 µl aliquot of the activated MP is then mixed with 100 µl reagent solution (2.5 mM 5,5'-dithio-bis-2-nitrobenzoic acid; DTNB, 100 µg/ml SBTI, 20 mM CaCl2 in the above buffer), and kept for 10 min at room temperature in order to react with all free SH-groups. The reaction is then started by adding 100 µl substrate solution (1.25 mM in buffer containing 100 µg/ml SBTI) and the changes in absorbance at 414 nm is measured 11 times at 1 min intervals.

### 3. Functional IL-1 Antagonistic Effects

The test method described in 2) above is repeated, but instead of adding human monocytes to the chondrocyte culture, IL-1 itself (recombinant human IL-1β, Sandoz) is added at a concentration of 1 ng/ml. MP activity in the chondrocyte culture medium is assayed after 2 days. Compounds of the invention are active as functional IL-1 antagonists at concentrations of 1-30 nM.

### 4. LPS-Fever

An LPS-suspension (Sigma, No. L-5886; 100 ug/5 ml glucose solution/kg s.c.) is injected in male Tuttlingen SD rats (150-160 g). Two hours later the body temperature is measured using a thermistor rectal probe connected to an ELLAB telethermometer. After 4 hr the test compound is administered. Two hours later (6 hr. after LPS administra- tion) the temperature is measured again. The temperature increment shown by the untreated controls is taken as 100% and that in the treated group is expressed as a percentage of this value. The ED50 is the dose causing a 50% inhibition of the temperature increase determined in the control rats. Compounds of the invention typically have an ED50 in this assay from about .1 to about 1 mg/kg.

### 5. Carrageenan-Induced Paw Edema in the Rat

Five OFA male rats, 150-170 g body weight, are used for each group. The test compound is administered orally as a suspension in physiological saline/0.5% tragacanth one hour prior to the carrageenan injection. Carrageenan (0.1 ml of a 1% suspension in physiological saline) is given by subplantar injection into one hind paw. The swelling of the paw is measured by means of an antiphlogometer according to Kemper & Amelm. A control reading is taken immediately after the injection, and the swelling is measured after 3 and 5 hrs. The mean value of the 3- and 5-hour reading is taken after deduction of the control reading, and the values obtained from the treated animals are expressed as a percentage of the value obtained from non-treated controls. The ED50 is the dose causing a 50% inhibition of the carrageenan-induced swelling after 3 hr. Compounds of the invention typically have ED50s in this assay of from about .1 to about 1 mg/kg.

The compounds of the invention are therefore indicated for the treatment of disorders with an aetiology associated with or comprising excessive cytokine release, particularly IL-1β release, e.g. in a wide variety of inflammatory states and diseases such as rheumatoid arthritis, osteoarthritis, septic shock, psoriasis, atherosclerosis, inflammatory bowel disease, Crohn's disease and asthma.

For the above uses the required dosage will of course vary depending on the mode of administration, the particular condition to be treated and the effect desired. In general however, satisfactory results are achieved at dosage rates of from about 0.1 to 20 mg/kg animal body weight, preferably 0.5 to 5 mg/kg. It has been determined that the compound of Formula I' has an IC₅₀ value in the THP-1 test (concentration of compound which inhibits to 50 % the release of IL-1β) of 0.06 µM. It is therefore indicated that this compound may be administered at daily dosages of from 8 mg to 2 g p.o. to larger mammals, for example humans.

In accordance with the foregoing the present invention also provides:
i) A method for the treatment of disorders with an aetiology associated with or comprising excessive cytokine release, particularly IL-1β release, such as rheumatoid arthritis, osteoarthritis, septic shock, psoriasis, atherosclerosis, inflammatory bowel disease, Crohn's disease and asthma, in a subject in need thereof, which method comprises administering to said subject an effective amount of
   a) a compound of Formula I as defined above; or
   b) a compound of formula II in which:
      R₁ is H or methyl;
      R₂ and R₃ are H or OH;
      R₄ is H or methoxy;
      -a-b- is -CH₂-CH₂- or -CH=CH-
      and c is C=O or CH-OH (other than compounds of formula I) ; or
   c) a compound of formula III in which R is H or methyl
      in free or base salt form, or a physiologically-hydrolysable and -acceptable ester thereof.
ii) A compound of formula II in which:
   R₁ is methyl; R₂ and R₃ are H or OH; R₄ is H or methoxy; -a-b- is -CH₂-CH₂- or cis- -CH=CH-; and c is C=O or CH-OH; in free base or salt form, or a physiologically-hydrolysable and -acceptable ester thereof for use as a pharmaceutical, for example for use in treating any of the diseases disclosed above.

The compounds of the invention may be administered by any conventional route, in particular nasally, enterally, preferably orally, e.g. in the form of tablets or capsules, or parenterally e.g. in the form of injectable solutions or suspensions or in a suppository form. Unit dosage forms contain, for example from about 2 mg to 1 g of the compound of the invention. The invention also provides a pharmaceutical composition comprising an effective amount of the compound of formula II as defined in b) above, in association with a pharmaceutically acceptable diluent or carrier. Such compositions may be manufactured in conventional manner.

The preferred compounds for pharmaceutical use are the compounds of formulae I', I'', X, XI, XII and XIII.

The following examples illustrate the invention:

### Examples

### Example 1: Fermentation example

A seed culture of strain NRRL 18919 is inoculated into a 2 l Erlenmeyer flask containing 1 l of sterile, liquid medium consisting of 2% malt extract and 0.4% yeast extract in de-ionized water. This Erlenmeyer flask is incubated on a rotary shaker at 200 rpm at 24°C for three days and serves as the preculture for a 50 liter fermentation. This intermediate culture is carried out in a 75 l fermenter containing 50 l of a sterile liquid medium consisting of 2% malt extract and 0.4% yeast extract in de-ionized water. During the fermentation the temperature is held constant at 25°C, the stirrer speed at 50 rpm, the aeration at 50 l/min and the overpressure at 0.5 bar. The preculture fermentation lasts for only one day after which it is used to inoculate a final fermentation at larger scale. This production culture is carried out in a 5000 l fermenter containing 3000 l of a sterile liquid medium composed of (g/l) 20 glucose, 2 soy-protein, 2 malt extract 2 yeast extract, 2 KH₂PO₄ and 2 MgSO₄ in de-ionized water. Prior to inoculation 1 mg/l Cyclosporin A dissolved in a small volume of methanol is added to the sterile medium. During the fermentation the temperature is held constant at 25°C, the stirrer speed at 25 rpm, the aeration at 3000 l/min and the overpressure at 0.5 bar. Exessive foaming is prevented by controlled addition of antifoam agent. The production of the compound of the invention is monitored by HPLC and the production culture is harvested at the time of highest titers of the desired metabolite.

### Example 2: Isolation of metabolite

The mycelium is separated from the culture broth and the supernatant (2800 1) is extracted with 2800 l of ethyl acetate. The mycelium is discarded. The ethyl acetate solution is evaporated to dryness under vacuum at 45-50°C. The compound of interest can be gained directly by crystalisation out of the extraction residue, by dissolving the extraction residue (ca. 1 kg) in 5 l of methanol at 60°C, cooling the resultant solution down suddenly and afterwards keeping it at room temperature for 20 hours. The resulting crystals (ca. 250 g) are separated by filtration, redissolved in methanol at 75°C and re-crystalised overnight at -25°C. The crystals are suction-filtred, washed with cold methanol and dried under vacuum. The product is [3S-(3R*, 5Z, 8R*, 9R*, 11E)]-3,4,9,10-tetrahydro-8,9,16-trihydroxy-13,14-dimethoxy-3-methyl-1H[2] benzoxacyclotetradecin-1,7[8H] dione, and is obtained in the form white needles having the following properties: m.p. 170-172°C, [α]_{D}²⁰ = -43.1° (c = 0.49 in methanol).

### Example 3: Preparation of the compound of formula I''

The compound of formula I'', [3S-(3R*, 5E, 8R*, 9R*, 11E)]-3,4,9,10-tetrahydro-8,9,16-trihydroxy-13,14-dimethoxy-3-methyl-1H-2-benzoxycyclotetra-decin-1,7 (8H)-dione, is prepared as follows.

The product of Example 2 (1 g) is dissolved in 100 ml pyridine and stirred under an argon atmosphere for 24 hr. at 50°C. The resulting yellow solution is concentrated under vacuum and the residue is taken up in ethyl acetate and shaken with 1N tartaric acid. The organic phase is separated, washed with brine, dried over sodium sulphate and evaporated. The solid residue is chromatographed on Kieselgel using ethyl acetate as mobile phase to give the compound of formula I'', m.p. 171-173°C. NMR (CDCl₃): 1.47 (d, 3H), 2.25-2.85 (m, 4H), 3.60 (s,3H), 3.89 (s,3H), 4.17 (m,1H), 4.65 (d,1H), 5.40 (m,1H), 5.93 (m,1H), 6.45 (s,1H), 6.47 (d,1H) 6.63 (dd,1H), 7.00 (dt,1H), 11.21 (s,1H) ppm.

### Example 4: Preparation of the compound of formula X

By analogy with Example 3, the compound of formula X, [3S-(3R*, 5E, 8R*, 9R*, 11E)]-3,4,9,10-tetrahydro-8,9,16 trihydroxy-14-methoxy-3-methyl-1H-2-benzoxacyclotetradecin-1,7(8H)-dione, is obtained.
M.p. 154-159°C.

### Example 5: Preparation of the compound of formula XI

By analogy with Example 3 the compound of formula XI, [3S-(3R*,5E,8R*,9R*,11E)]-3,4,9,10-tetrahydro-8,9-dihydroxy-13,14,16-trimethoxy-3-methyl-1H-2-benzoxacyclotetradecin-1,7(8H)-dione, is obtained as a foam having the following NMR spectrum. NMR (CDCl₃):1.4(d,3H); 2.2-3.0(m, 5H); 3.61(s,3H); 3.78(s,3H); 3.83(s,3H); 3.7-4.15 (m,2H); 4.75(d,3H); 2.2-3.0 (m,lH); 6.15(d,lH); 6.31(d,lH); 6.38(s,1H); 7.85(m,1H)ppm.

### Example 6: Preparation of the compound of formula XIII

a). Pent-1-en-5-one
   10 g (100 mMol) of 5-hydroxypent-1-ene in 100 ml of dichloromethane is added to a suspension of 43 g of pyridinium chlorochromate in dichloromethane at room temperature. After 1 1/2 hr diethylether is added, the suspension is filtered over silica gel, the solid residue washed several times with diethylether which is also filtered over silica gel, and the diethylether fractions are combined and evaporated slowly to yield 7. g of crude pent-1-en-5-one.
b). 4-Trimethylsiloxyhex-1-yne
   A mixture of 8.1 g of 4-hydroxyhex-1-yne, 16 g of t.butyldimethylsilyl chloride and 7.2 g of imidazole in 100 ml of acetonitrile is stirred overnight at room temperature, after which diethylether is added and the suspension filtered over silica gel. The filtrate solution is then evaporated and the residue distilled under water pump vacuum at 70°C to give 17.8 g (93%) of the title product.
c). 2-Trimethylsiloxy-7-hydroxy-undeca-4-yn-10-ene
   A solution (37 ml) of butyl lithium is added dropwise at -78°C to a solution of 13.8 g of the product of part (b) above in 20 ml of tetrahydrofuran and the mixture is stirred at -78°C for 1 1/2 hr. A solution of 6.0 g of the aldehyde product of part a) above in 10 ml of tetrahydrofuran is then added slowly, the mixture stirred at -78°C for 1 hr and then allowed to warm to room temperature. Water is then added, the mixture extracted with ethyl acetate and the organic phase dried, filtered and evaporated to give 13.1 g (72%) of the title product in impure form.
d). 2-Trimethylsiloxy-7-hydroxy-undeca-cis-4,10-diene
   To a solution of 10.1 g of the product of part c) above in 300 ml of pyridine is added 1 g of 10% palladium in BaSO₄ and the mixture is hydrogenated at room temperature. After 24 hr 420 ml of hydrogen is taken up and after a further 24 hr 720 ml of hydrogen is taken up. The mixture is then suction filtered over Hyflo to remove the catalyst, the product concentrated, dissolved in ethyl acetate, washed with 10% citric acid solution and saturated brine, dried over Na₂SO₄ and concentrated. At this point, however, thin layer chromatography indicates that only 15% of the starting material has been converted. The product is, therefore redissolved in 300 ml of pyridine and 1.5 of 10% palladium in BaSO₄ is added and the mixture hydrogenated with stirring at room temperature. It is now found that already after 6 hr 700 ml of hydrogen has been taken up. The product (10.2 g) is found to be about 90% converted as judged by thin layer chromatography.
e). 2-Trimethylsiloxy-7-{(2-methoxy)ethoxy}methoxy-undeca-cis-4,10-diene
   10 g of the title product of step d) above is dissolved in 80 ml of dichloromethane with stirring at room temperature and 8.5 ml of diisopropylethylamine is added dropwise over 1 min followed by 5.7 ml of 1-chloromethyl-2-methyl-glycol in 20 ml of dichloromethane dropwise over 10 min. After 2 hr the reaction is complete as determined by thin layer chromatography. The reaction mixture is washed with 10% NaHCO₃, 10% citric acid solution and saturated brine, dried over Na₂SO₄ and concentrated to 14 g of the title product in rude form. The product is purified by silica gel chromatography (hexane/diethylether 8:1)with a yield of 10.5 g (83%).
f). The compound of formula IV in which R₄ is H, R₅ and R₆ are MeO, -a-b- is cis- -CH=CH-, -d-e- is -CH₂-CH₂ and -f-g- is trans -CH=CH-
   90 ml of triethylamine is stirred under an atmosphere of argon and to this are added 7.5 g of a compound of formula V ,in which R₄ is H, R₅ and R₆ are MeO and R₁₂ is Me, followed by 10.8 g of the product obtained in step e) above, 0.104 g of palladium diacetate, 0.564 g of tri o-tolyl phosphine and 3.9 g of silver acetate. The mixture is warmed to 80°C with stirring in an oil bath and after 60 hr a further 0.104 g of palladium diacetate and 0.564 g of tri o-tolyl phosphine is added and the reaction continued for a further 48 hr at which time the reaction is complete as judged by thin layer chromatography. The product mixture is concentrated, treated with ethyl acetate and removed from the undissolved residue by suction over Hyflo. The liquid is concentrated and the intermediate trimethylsiloxy/R₁₂ is Me product purified by silica gel chromatography (hexane/ethyl acetate 4:1) to give a yield of about 14 g.
   5.1 g of NaOH is dissolved in 50 ml of DMSO and 30 ml of water, 7.44 g of the above intermediate product is added and the mixture heated at 100°C for 4 hr with stirring in an oil bath. The mixture is then cooled, diluted with water, washed 3 times with ether, cooled with ice and adjusted to pH3 with 2N HCl. The product is then extracted 3 times with ethyl acetate, washed twice with saturated brine, dried over Na₂SO₄, concentrated and dried under high vacuum to give 6 g of the title product (m/z: 453 (MH+), 348(60), 329(100), 285(60), 207(60).
g). The compound of formula XIII
   5.4 g of the product of step f) above is added to 3.5 l of tetrahydrofuran at room temperature with stirring, 18.7 ml of diethyl azodicarboxylate is added followed by 31.3 g of triphenylphosphine and the mixture warmed to 50°C in an oil bath and allowed to react overnight. The product mixture is concentrated and the residue is purified using 2 Al₂O₃ columns and elution with hexane/ethyl acetate (2:1). Fractions 6-1 from the first column and fractions 7-20 from the second column together yield 5 g of crude product, which is further purified by silica gel chromatography to yield 3.2 g (62%) of the pure resinous intermediate product, being the product corresponding to the compound of formula XIII in which c is in the CH-OCH₂-O-CH₂-CH₂-OCH₃ protected form.
   This intermediate product is converted to the further intermediate product in which c is CHOH by treatment with acid. 5.2 g of the above intermediate product is dissolved in 12 ml of tetrahydrofuran, 10 ml of IN HCl is added and the mixture stirred at 45°C for 24 hr after which it is extracted three times with ethyl acetate and washed with saturated brine and NaHCO₃ solutions. The ethyl acetate phases are dried over Na₂SO₄, concentrated and purified by silica gel chromatography (hexane/ethyl acetate 1:1) to give about 3.4 g of the sticky further intermediate product.
   2 ml of dichloromethane is stirred at room temperature, 76 µl of oxalyl chloride is added, the mixture cooled to -60°C and a solution of 0.13 ml of DMSO in 1 ml of dichloromethane is added dropwise over 3 min. The resultant evolution of gas is allowed to proceed for 3 min after which a solution of 280 mg of the above further intermediate product in 3 ml of dichloromethane is added dropwise over 5 min with formation of a precipitate. The mixture is stirred for a further 15 min after which 0.56 ml of triethylamine is added over a 5 min period with the resultant precipitation of a paste which is allowed to accumulate at room temperature. The paste is extracted with dichloromethane, the organic phases washed twice with 10% citric acid twice with saturated brine solution, dried over Na₂SO₄, concentrated and purified by silica gel chromatography (dichloromethane) to give the title product of formula XIII in pure form. The pure product has the following characteristics: m. pt.159°C; m/z: 344(M+), 217(80), 189(60), 178(100), 95(60).

### Example 7: Preparation of the compound of formula XII

a). The analogue of the compound of formula XII in which c is CH-OCH₂-O-CH₂-CH₂-OCH₃
   2.9 g of Iodine and 0.55 g of magnesium are added to 20 ml of diethyl ether and stirred until the exothermic reaction is completed and the brown colour has disappeared. By means of a syringe 11 g of the resultant solution is removed and added dropwise over a period of 10 min to a solution in benzene of 1.64 g of the first intermediate product of step g) of Example 6 above. A yellow precipitate forms and the mixture is warmed to 80°C in an oil bath and stirred for 1 1/2 hr at this temperature and then stirred overnight at room temperature. The product is then extracted twice with ethyl acetate, washed with NaHCO₃ and saturated brine, dried over Na₂SO₄, concentrated and purified by silica gel chromatography (hexane/ethyl acetate 7:3) to give 1.6 g of the title product in pure form.
b). The analogue of the compound of formula XII in which c is CHOH
   510 mg of the product of step a) of this Example is dissolved in 12 ml of tetrahydrofuran, 10 ml of 1 N HCl is added and the mixture stirred at 45°C for 24 hr. The product mixture is extracted three times with ethyl acetate, and washed with saturated brine and NaHCO₃. The ethyl acetate phases are dried over Na₂SO₄, concentrated and purified by silica gel chromatography (hexane/ethyl acetate 1:1) to give 330 mg of the sticky title product (m/z: 332(M+), 314(10), 203(75), 190(55), 164(60).
c). The compound of formula XII
   280 mg of the product of step b) is dissolved in 10 ml of dichloromethane with stirring at room temperature, 475 mg of pyridinium dichromate is added and the mixture is stirred overnight at room temperature. After this a further 158 mg of pyridinium dichromate is added and the mixture is stirred again overnight and for a further 24 hr at room temperature. The resultant product solution is separated from the solid byproducts by filtration through Hyflo and purified chromatographically (elution with dichloromethane) to yield the title product in pure form (m. pt. 137°C; m/z: 330(M+), 312(20), 202(100), 175(75))

### Example 8: The compound of formula I in which R₄ is H, R₅ and R₆ are OMe, -a-b- is cis -CH=CH-, c is C=O and -d-e- and -f-g- are -CH₂-CH₂-

a). 4-dimethyl,t-butyl-silyloxy-pent-1-yne
   17.8 g of (±)-4-pentyn-2-ol is dissolved in 150 ml of acetonitrile with stirring at room temperature, 15.8 g of imidazole followed by 35 g of dimethyl,t butyl-silyl chloride are added giving rise to an exothermic reaction whereby all components of the reaction mixture initially go into solution but afterwards a precipitate immediately forms. The mixture is stirred overnight at room temperature after which it is suction filtered, the liquid fraction concentrated and a further 2.9 g of imidazole and 6.3 g of dimethyl,t butyl-silyl chloride are added. After 3 hr the reaction mixture is again suction filtered, the liquid fraction concentrated subjected to fractional distillation. The title product is in the first three fractions which are combined and the product purified by distillation at reduced pressure (12 mm Hg) at 64-68°C.
b). The product of formula VII in which R₄ is H, R₅ and R₆ are OMe and R₁₂ is Me
   14.6 g of the corresponding compound of formula V, 14.6 g of the product of step a) of this Example, 0.3 g of palladium diacetate, 1.1 g of tri o-tolyl phosphine and 7.6 g of silver acetate are added to 60 ml of tetrahydrofuran and the mixture warmed with stirring to 70°C in an oil bath. After 48 hr, at which time the reaction is about 70% completed as judged by thin layer chromatography, a further 0.3 g of palladium diacetate and 1.1 g of tri o-tolyl phosphine are added and the mixture warmed over the weekend at 70°C. The mixture is then suction filtered over Hyflo, concentrated under high vacuum and purified by silica gel chromatography (hexane/ethyl acetate 4:1) to give a first intermediate product, i.e. an analogue of the corresponding compound of formula VII in which R₆ = OMe, R₅ = OMe, R₄ = H, R₁₂ = Me and the aldehyde group of the C₆ side chain is a dimethyl,t-butyl-silyl-protected OH group.
   6.7 g of this first intermediate product is dissolved in 100 ml of methanol, 500 mg of palladium/charcoal catalyst containing 10% palladium (Pd/C 10%) is added and the mixture hydrogenated under stirring for 48 hr (350 ml of H₂ being taken up after the first 24 hr). The reaction mixture is then suction filtered over Hyflo and concentrated; though at this stage it is found that the C₆ side chain double bond is only 30% reduced as judged by NMR. The reaction product is, therefore again taken up in 150 ml of methanol, 500 mg of the Pd/C 10% catalyst is added and the mixture hydrogenated over the weekend at room temperature. The resultant reaction mixture is suction filtered over Hyflo, concentrated and a second intermediate product, i.e. an analogue of the corresponding compound of formula VII in which the aldehyde group of the C₆ side chain is an OH group, is separated from the corresponding siloxy-protected compound by silica gel chromatography (hexane/ethyl acetate 2:1) in a yield of 4.9 g.
   1.2 ml of oxalyl chloride is added to 30 ml of dichloromethane stirred at room temperature, the mixture cooled to -60°C and 1.9 ml of DMSO in 7 ml of dichloromethane added dropwise over a 5 min period after which the mixture is stirred for a further 3 min and a solution of 3.5 g of the second intermediate product of this example in 20 ml of dichloromethane is added dropwise over a 10 min period. A precipitate forms, the mixture is stirred for a further 20 min, concentrated and 8.2 ml of triethylamine is added over a 5 min period. A thick paste precipitate forms and is allowed to accumulate at room temperature for 1.5 hr. The solid product is extracted with dichloromethane, washed twice with 10% citric acid solution, once each with saturated brine and NaHCO₃, dried over Na₂SO₄ and concentrated to give 3.6 g of the title product in the form of gum.
c). The product of formula IV in which in which R₄ is H, R₅ and R₆ are OMe, -a-b- is cis -CH=CH-, -d-e- and -f-g- are -OH₂-OH₂- and R₁₁ is H
   0.39 g of the product of step a) of this Example is added to 5 ml of tetrahydrofuran under an atmosphere of nitrogen, 1.2 ml of butyl lithium is added dropwise over a 2 min period at -70°C. After 1 hr a solution of 0.5 g of the final product of step b) of this Example in 4 ml of tetrahydrofuran is added dropwise over a 5 min period, the mixture stirred at -70°C for 1.5 hr and then allowed to warm to room temperature. At this point the reaction is complete as judged by thin layer chromatography. Whilst cooling with ice, the product is washed with 10% citric acid solution, extracted three times with ethyl acetate, washed with saturated brine and NaHCO₃, dried over Na₂SO₄ and concentrated to give 0.87 g of a resinous first intermediate product, i.e. an analogue of the corresponding compound of formula IV in which the COOH substituent is in the form of the methoxy ester, the bond corresponding to the -a-b- bond of the compound of formula I is an acetylene bond and the OH substituent on the C₁₀ carbon atom of the C₁₁ side chain is t-butyl-silyl-protected.
   3.1 g of this first intermediate product is dissolved in 50 ml of pyridine, 200 mg of 10% Pd/BaSO₄ is added and the mixture hydrogenated at room temperature with magnetic stirring. After 4 hr greater than the theoretical hydrogen requirement (240 ml) has been taken up. The resultant product is then sampled, concentrated, taken up in dichloromethane and washed with 10% citric acid solution (at this point the reaction is judged to be complete by NMR). The product is separated from the catalyst by suction filtration over Hyflo, concentrated, taken up in ethyl acetate, washed with saturated brine, dried over Na₂SO₄ and concentrated to give 3.05 g of a resinous second intermediate product, i.e. the analogue of the first intermediate product of this step in which the bond corresponding to the -a-b- bond of the compound of formula I is cis -CH=CH-.
   By analogy with step f) of Example 6 the second intermediate product of this step is converted into the title product (m/z: 367(MH+), 349(40), 331(100), 305(90), 191(100)).
d). The compound of formula I in which R₄ is H, R₅ and R₆ are OMe, -a-b- is cis -CH=CH- c is C=O and -d-e- and -f-g- are -CH₂-CH₂-
   1.7 g of the final product of step c) above is dissolved with stirring in 1.8 1 of acetonitrile, 5.9 g of 2-chloropyridine iodide and 6.5 ml of triethylamine are added and the mixture is warmed over the weekend to 50°C in an oil bath with stirring. The product is then concentrated, taken up in ethyl acetate, washed with 10% citric acid solution NaHCO₃ and saturated brine, dried over Na₂SO₄ and concentrated to give 1.6 g of a crude intermediate product (i.e. the analogue of the title product in which c is CHOH) in the form of a brown resin. This crude product is purified by silica gel chromatography (hexane/ethyl acetate 3:2), using dichloromethane to dissolve the intermediate product.
   To 2 ml of dichloromethane at room temperature is added 5 µl of oxalyl chloride, the mixture cooled to -70°C and a solution of 93 µlof DMSO in 1 ml of dichloromethane is added dropwise over 3 min with stirring. The mixture is stirred for a further 5 min after which a solution of 0.2 g of the intermediate product of this step is added dropwise over 5 min. A precipitate forms, the mixture is stirred for a further 15 min, 0.4 ml of triethylamine is added dropwise over 5 min, the mixture stirred for 1 hr at -70°C and then allowed to warm to room temperature over 1 hr. The resultant product mixture is diluted with dichloromethane, washed twice with 10% citric acid solution, once with saturated brine, dried over Na₂SO₄, concentrated and purified by silica gel chromatography and recrystallisation to give about 100 mg of pure product (m. pt. 110°C).

### Example 9: The compound of formula I in which R₄ and R₆ are H, R₅ is OMe, -a-b- is trans -CH=CH- c is C=O and -d-e- and -f-g- are -CH₂-CH₂-

35 mg of magnesium is stirred with 1 ml of diethylether and 1 ml of benzene and 0.18 g of iodine is added, the dark brown colour dissipating after 1.5 hr. The liquid phase of the resultant mixture is added to a solution of 50 mg of the final product of Example 6 in 2 ml of benzene with the resultant formation of a precipitate. The mixture is stirred at 60°C for 2.5 hr, the product taken up in ethyl acetate, washed with 1N HCl saturated NaHCO₃ and saturated brine, dried over Na₂SO₄, concentrated and purified by silica gel chromatography (toluene/methanol 98:2). The product is further purified on a silica gel column (hexane/ethyl acetate 3:2) to give about 30 mg of the pure title product in the form of an oil (m/z: 333(MH+), 315(80), 265(100), 177(90).

### Example 10: The compound of formula I in which R₄ is H, R₅ and R₆ are OMe, -a-b- is trans -CH=CH- c is C=O and -d-e- and -f-g- are -CH₂-CH₂-

By analogy with Examples 3 and 9 the title product is prepared form the corresponding product in which -a-b- is cis -CH=CH-, i.e. the final product of Example by isomerisation of the -a-b- bond. The title product is obtained in the form of an oil (m/z: 346(M+), 205(50), 191(100), 178(40), 152(40, 95(55).

## Claims

1. A compound of Formula I 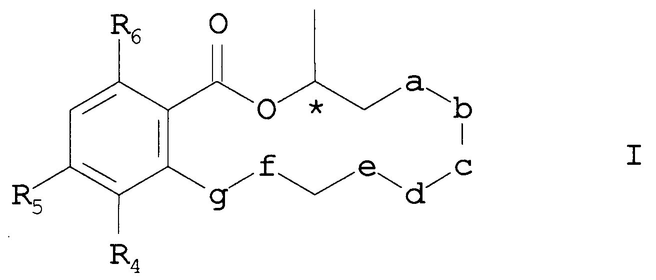 in which R₄ and R₆ are the same or different and are H, OH, C₁₋₄ alkoxy or C₁₋₄alkyl COO-,
R₅ is OH, C₁₋₄alkxoy or C₁₋₄alkyl COO-,
one of -a-b- or -d-e- is -CHR₇-CHR₈- and the other is cis- or trans- -CR₇=CR₈-, wherein R₇ and R₈ are the same or different and are H, OH, C₁₋₄ alkoxy or C₁₋₄alkyl COO-,
c is CH-OH or C=O and
-f-g- is -CH₂-CH₂- or cis- or trans- -CH=CH-
provided that, when R₄ is H, R₆ is OH and -f-g- is trans- -CH=CH-; 1. R₅ is not OH when -a-b- is -CH₂-CH₂-, c is C=O and -d-e- is -CH₂-CH₂-, or 2. R₅ is not methoxy when -a-b- is -CH₂-CH₂- or cis- -CH=CH- and c is C=O or CH-OH and -d-e- is -CHOH-CHOH-,
in free form or base salt form or in the form of a physiologically-hydrolysable and -acceptable ester and wherein the asymmetric carbon marked * and the atoms a and/or b or d and/or e, when these are asymmetric carbon atoms have the R- or S-configuration or the compound comprises any mixture of the optical isomers thereof.

2. A compound according to claim 1 in which
R₄ and R₆ are the same or different and are H, -OH, MeO- or Me-COO-, R₅ is -OH, MeO- or MeCOO-, -a-b- is cis- or trans- -CR₇'=CR₈'-, wherein R₇' and R₈' are the same or different and are H, OH, MeO- or Me-COO- and -d-e- is -CHR₇'-CHR₈'-, wherein R₇' and R₈' are as defined.

3. A compound according to claim 2, in which
R₄ is H or MeO, R₅ is MeO, R₆ is OH or MeO, -a-b- is cis- or trans- -CH=CH-, -d-e- is -CH₂-CH₂- or -CHOH-CHOH- and -f-g- is trans- -CH=CH-.

4. A compound of formula 1 in which
R₄ is H or methoxy, R₅ is methoxy, R₆ is OH, -a-b- is cis- or trans- -CH=CH-, c is CHOH or C=O, -d-e- is -CHOH-CHOH- and -f-g- is trans- -CH=CH-; provided that when -a-b- is cis -OH=OH-, then R₄ is methoxy and c is C=O in free form or base salt form or in the form of a physiologically-hydrolysable and -acceptable ester.

5. A compound according to any of the preceding claims, in which a-b is trans- -CH=CH-.

6. A compound according to any of the preceding claims, in which the asymmetric carbon atoms all have the S-configuration.

7. A compound of formula I', I'', X, XI, XII, or XIII as hereinbefore defined.

8. A process for the preparation of the compound of formula I' comprising the steps of cultivating an 87-250904-F1-producing fungal strain and isolating the metabolite 87-250904-F1 which is formed.

9. A process according to Claim 8 in which the fungal strain is NRRL 18919.

10. A pure culture of fungal strain NRRL 18919.

11. A fermentation broth which is obtained by cultivation of the fungal strain NRRL 18919.

12. A process for the preparation of a compound of formula I in which a-b is trans- -CH=CH- comprising the step of isomerization of the corresponding cis- isomer under mild alkaline conditions.

13. A process for the preparation of a compound of formula I, in which c is CHOH, which comprises cyclising a compound of formula IV 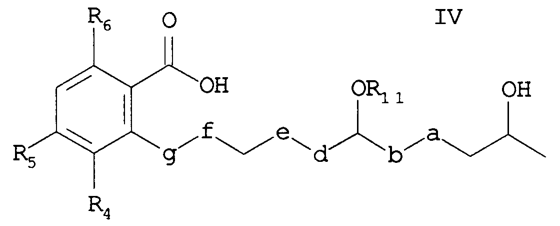 wherein R₄, R₅, R₆, -a-b-, -d-e- and -f-g- are as defined in claim 1 with the exception that any OH substituents on -a-b- or -d-e- are in suitably protected form, and R₁₁ is H or an OH protecting group;
and removing any OH protecting groups therefrom.

14. A process for the preparation of a compound of formula I, in which c is C=O comprising oxidising the c CHOH group of a compound of formula I wherein c is CHOH.

15. A method for the treatment of disorders with an aetiology associated with or comprising excessive cytokine release, particularly IL-1β release, such as rheumatoid arthritis, osteoarthritis, septic shock, psoriasis, atherosclerosis, inflammatory bowel disease, Crohn's disease and asthma, in a subject in need thereof, which method comprises administering to said subject an effective amount of
a) a compound of Claim 1; or
b) a compound of formula II 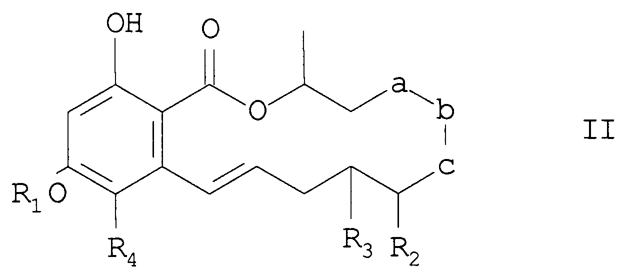 in which:
R₁ is H or methyl;
R₂ and R₃ are H or OH,
R₄ is H or methoxy;
-a-b- is -CH₂-CH₂- or -CH=CH-
and c is C=O or CH-OH (other than a compound of Claim 1); or
c) a compound of formula III 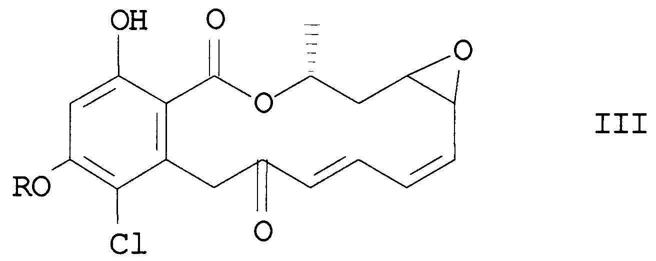 in which R is H or methyl
in free or base salt form, or a physiologically-hydrolysable and -acceptable ester thereof.

16. A compound of formula II, stated in Claim 15, in which:
R₁ is methyl;
R₂ and R₃ are H or OH,
R₄ is H or methoxy;
-a-b- is -CH₂-CH₂- or cis- -CH=CH-;
and c is C=O or CH-OH;
in free base or salt form, or a physiologically-hydrolysable and -acceptable ester thereof for use as a pharmaceutical.

17. A pharmaceutical composition comprising a compound according to claim 1.

18. A pharmaceutical composition comprising a compound of formula II, stated in Claim 15, in which:
R₁ is methyl; R₂ and R₃ are H or OH; R₄ is H or methoxy;-a-b- is -CH₂-CH₂- or cis- -CH=CH-; and c is C=O or CH-OH; in free base or salt form, or a physiologically-hydrolysable and -acceptable ester thereof.
